(19) Europäisches Patentamt
European Patent Office
Office européen des brevets

(11) Veröffentlichungsnummer : **0 165 322**
**B1**

(12) # EUROPÄISCHE PATENTSCHRIFT

(45) Veröffentlichungstag der Patentschrift : **20.01.88**

(51) Int. Cl.⁴ : **C 07 C121/75**, C 07 C121/66

(21) Anmeldenummer : **84106868.7**

(22) Anmeldetag : **15.06.84**

(54) **Verfahren zur Herstellung basisch substituierter Phenylacetonitrile.**

(43) Veröffentlichungstag der Anmeldung : **27.12.85 Patentblatt 85/52**

(45) Bekanntmachung des Hinweises auf die Patenterteilung : **20.01.88 Patentblatt 88/03**

(84) Benannte Vertragsstaaten : **AT BE CH DE FR GB IT LI LU NL SE**

(56) Entgegenhaltungen :
**EP-A- 0 047 888**
**FR-A- 1 553 708**
**THE CHEMISTRY OF THE CYANO GROUP, 1970, ausgegeben von ZVI Rappoport, Seite 308:A, Interscience Publishers, London, GB;**

(73) Patentinhaber : **LUDWIG HEUMANN & CO GMBH**
**Heideloffstrasse 18-28 Postfach 2260**
**D-8500 Nürnberg (DE)**

(72) Erfinder : **Kisielowski, Lothar, Dr.**
**Meuschelstrasse 1**
**D-8500 Nürnberg 10 (DE)**
Erfinder : **Grafe, Ingomar, Dr.**
**Auerbacher Strasse 16**
**D-8500 Nürnberg (DE)**
Erfinder : **Liebenow, Walter, Dr.**
**Ganghoferstrasse 23**
**D-8500 Nürnberg (DE)**
Erfinder : **Ahrens, Kurt Henning, Dr.**
**Praterstrasse 9**
**D-8500 Nürnberg (DE)**

(74) Vertreter : **Kraus, Walter, Dr. et al**
**Patentanwälte Kraus, Weisert & Partner Thomas-Wimmer-Ring 15**
**D-8000 München 22 (DE)**

**EP 0 165 322 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung basisch substituierter Acetonitrile.

Es ist bekannt, daß basisch substituierte Phenylacetonitrile coronarvasodilatorische und antiarrhythmische Eigenschaften besitzen und daher wertvolle Arzneimittel zur Behandlung verschiedener Koronarkrankheiten sind. Die bekannteste Substanz ist das α-Isopropyl-α-[(N-Methyl-N-homoveratryl)-γ-aminopropyl]-3,4-dimethoxyphenylacetonitril (Verapamil, DE-PS 1 154 810).

Aus den DE-PS 11 54 810, DE-PS 11 58 083, DE-OS 20 59 923, DDR-PS 119 579 und DE-OS 31 21 766 sind bereits verschiedene Verfahren zur Herstellung solcher basisch substituierter Phenylacetonitrile bekannt.

Bei dem in der DE-PS 11 54 810 beschriebenen Verfahen geht man zur Herstellung der bekanntesten Verbindung dieser Substanzklasse, dem Verapamil, vom α-Isopropylveratrylcyanid der Formel

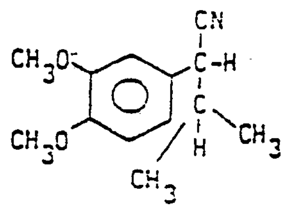

aus. Ausgangsmaterial dieser Verbindung ist das 3,4-Dimethoxybenzylcyanid. Nach Organikum, VEB Deutscher Verlag der Wissenschaften, Berlin 1976, Seiten 412 und 270 und DDR-PS 94 51 wird diese Verbindung aus Veratrol und Formaldehyd/wäßrige HCl und Umsetzung der Chlormethylverbindung mit NaCN hergestellt. Bei der Nacharbeitung des in der Literatur angegebenen Verfahrens zur Darstellung des 3,4-Dimethoxybenzylcyanids konnten in keinem Fall die in der Literatur angegebenen Ausbeuten erhalten werden. Es wurden lediglich geringe Mengen an 3,4-Dimethoxybenzylchlorid erhalten, die keine wirtschaftliche Herstellung von α-Isopropylveratrylcyanid ermöglichten.

Auch sind diese Verfahren technisch nicht anwendbar, da es schwierig ist, das Zwischenprodukt der Formel

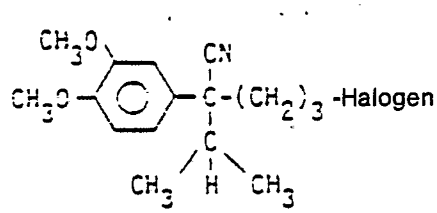

in guter Reinheit und Ausbeute herzustellen.

Weiter wird in der DE-OS 22 63 527 ein Verfahren zur Herstellung von Verapamil angegeben, bei dem α-Isopropylveratrylcyanid mit 3-Methyl-formamido-1-chlorpropan zum 2-(3,4-Dimethoxyphenyl)-2-isopropyl-5-methylformamidovaleronitril umgesetzt wird, das nach Abspaltung des Ameisensäurerestes mit Homoveratrylaldehyd kondensiert wird. Auch dieses Verfahren verläuft mit schlechten Ausbeuten.

In der DE-AS 26 31 222 ist ein Verfahren zur Herstellung basisch substituierter Phenylacetonitrile beschrieben, bei dem man zunächst ein α-Isopropylbenzylcyanid mit einem ω-Halogenacetal kondensiert, dann das erhaltene Nitrilacetal mit wäßriger Säure in den Nitrilaldehyd überführt und diesen mit einem Amin einer hydrierenden Kondensation unterwirft.

Bei allen diesen Verfahren ist die Herstellung der Vorprodukte mit Schwierigkeiten verbunden, und die Verfahren liefern daher das gewünschte Produkt nur mit geringer Ausbeute.

Demgegenüber ist es Aufgabe der Erfindung, ein neues und vereinfachtes Verfahren zur Herstellung von Verapamil und verwandten Verbindungen zur Verfügung zu stellen.

Diese Aufgabe wird erfindungsgemäß durch ein Verfahren zur Herstellung basisch substituierter Phenylacetonitrile der allgemeinen Formel I

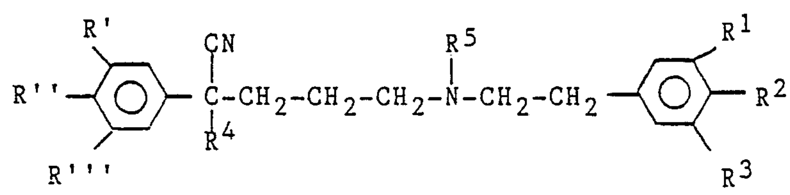

(I)

in der R′, R″, R‴, $R^1$, $R^2$ und $R^3$ unabhängig voneinander jeweils ein Wasserstoffatom, ein Halogenatom, eine Niedrigalkylgruppe, eine Niedrigalkoxygruppe, eine Niedrigalkylmercaptogruppe oder eine gegebenenfalls durch eine Niedrigalkylgruppe mono- oder disubstituierte Aminogruppe bedeuten, $R^4$ eine lineare oder verzweigte aliphatische Kohlenwasserstoffgruppe mit 1 bis 6 Kohlenstoffatomen, eine gesättigte oder ungesättigte cyclische Kohlenwasserstoffgruppe mit 5 oder 6 Kohlenstoffatomen bedeutet und $R^5$ für ein Wasserstoffatom oder eine Niedrigalkylgruppe steht, gelöst, das dadurch gekennzeichnet ist, daß man eine Verbindung der allgemeinen Formel II

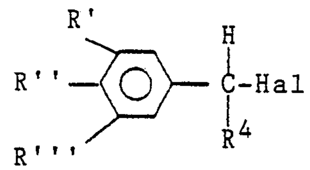

(II)

in der R′, R″, R‴ und $R^4$ die oben angegebenen Bedeutungen haben und Hal für ein Halogenatom steht, mit einem Cyanid der Formel Me-CN, worin Me ein einwertiges Metall bedeutet, zum Nitril der Formel III

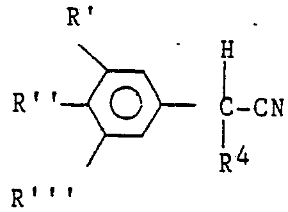

(III)

worin R′, R″, R‴ und $R^4$ die oben angegebenen Bedeutungen haben, umsetzt, die erhaltene Verbindung III mit einem β-substituierten Propionitril der allgemeinen Formel IV

$$NC—CH_2—CH_2—Y \qquad (IV)$$

in der Y eine Niedrigalkoxygruppe oder die Gruppe NH-Z, worin Z für eine Niedrigalkylgruppe eine $C_5$-$C_6$-Cycloalkylgruppe oder einen gegebenenfalls substituierten Phenylrest steht, bedeutet, umsetzt und die erhaltene Verbindung V

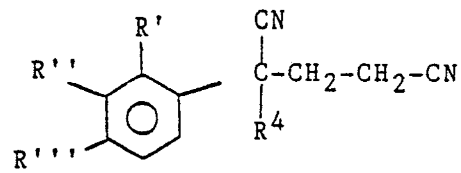

(V)

in der R′, R″, R‴ und $R^4$ die oben angegebenen Bedeutungen haben, in Gegenwart eines Phenylethylamins der Formel VI

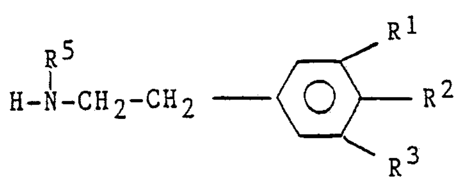

(VI)

in der $R^1$, $R^2$, $R^3$ und $R^5$ die oben angegebenen Bedeutungen haben, reduziert und gegebenenfalls die erhaltene Substanz mit einer physiologisch verträglichen Säure in ihr pharmakologisch annehmbares Salz umwandelt.

In der allgemeinen Formel I stehen R′, R″, R‴, $R^1$, $R^2$ und $R^3$ unabhängig voneinander jeweils für ein Wasserstoffatom, ein Halogenatom, zum Beispiel ein Fluor-, Chlor-, Brom- oder Jodatom, eine Niedrigalkoxygruppe, zum Beispiel eine Butoxy-, n- oder i-Propoxy-, Ethoxy- oder Methoxygruppe, eine Niedrigalkylmercaptogruppe, zum Beispiel eine Ethyl- oder Methylmercaptogruppe, oder eine Aminogruppe, die durch eine Niedrigalkylgruppe mono- oder disubstituiert sein kann. Hierin wird unter « Niedrigalkylgruppe » eine Alkylgruppe mit 1 bis 4 Kohlenstoffatomen verstanden. Unter « Niedrigalkoxygruppe » wird eine Alkoxygruppe mit 1 bis 4 Kohlenstoffatomen im Alkylteil verstanden. $R^4$ bedeutet eine lineare oder verzweigte aliphatische Kohlenwasserstoffgruppe mit 1 bis 6 Kohlenstoffatomen, vorzugsweise eine Niedrigalkylgruppe, insbesondere eine Methylgruppe, oder einen gesättigten oder ungesättigten

cyclischen Kohlenwasserstoff mit 5 oder 6 Kohlenstoffatomen, zum Beispiel eine Cyclopentyl- oder Cyclohexylgruppe oder eine Cyclopentenyl- oder Cyclohexenylgruppe. $R^5$ bedeutet ein Wasserstoffatom oder eine Niedrigalkylgruppe, wie eine n- oder i-Butyl-, n- oder i-Propyl-, Ethyl- oder Methylgruppe, wobei letztere bevorzugt wird. Bei dem in der ersten Stufe verwendeten Metallcyanid Me-CN bedeutet Me ein einwertiges Metall, beispielsweise Kalium oder Natrium, wobei letzteres bevorzugt wird. In der Formel IV steht Y für eine Niedrigalkoxygruppe, wie oben definiert, oder die Gruppe NH-Z, worin Z für Niedrigalkyl, $C_5$-$C_6$-Cycloalkyl oder gegebenenfalls substituiertes Phenyl steht. Als Substituenten der Phenylgruppe kommen zum Beispiel Halogenatome oder Niedrigalkylgruppen in Betracht.

In der ersten Stufe des erfindungsgemäßen Verfahrens wird die Verbindung der Formel II mit dem Metallcyanid umgesetzt. Die Verbindung II kann leicht aus dem entsprechenden Alkohol und der entsprechenden wäßrigen Halogenwasserstoffsäure hergestellt werden. Die Umsetzung erfolgt bei Temperaturen von 10 bis 40 °C und vorzugsweise mit wäßriger Salzsäure. So kann man zum Beispiel α-Isopropylveratrylalkohol der Formel

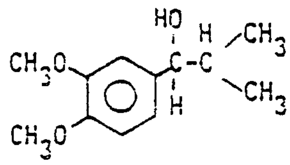

mit wäßriger Salzsäure zu der entsprechenden Verbindung II umsetzen. Die Verbindung II wird mit einem Metallcyanid, vorzugsweise Natriumcyanid, in Gegenwart eines Phasentransferkatalysators, wie Cetyltrimethylammonium- oder Tetrabutylammoniumsalz und 1 bis 10 Gew.-% Wasser, zum Nitril der Formel III umgesetzt. Der Phasentransferkatalysator wird vorzugsweise in einer Menge von 1 bis 20 Mol-% eingesetzt. Die Umsetzung erfolgt vorzugsweise im basischen Medium, vorzugsweise in einem Amin, wie Triethylamin, und bei einer Temperatur von 30 bis 80 °C. Wie bereits erwähnt, wird die Umsetzung in Gegenwart eines Phasentransferkatalysators durchgeführt. Die Umsetzung bei Phasentransferbedingungen erfolgt nach den allgemeinen Methoden, beschrieben in Synthesis, 1973, Seiten 441 und 447.

In der nächsten Stufe des erfindungsgemäßen Verfahrens wird die in der ersten Stufe erhaltene Verbindung III mit einem β-substituierten Propionitril der allgemeinen Formel IV umgesetzt. Die Umsetzung erfolgt in einem inerten Lösungsmittel, beispielsweise Dimethylformamid, und bei einer Temperatur von 40 bis 90 °C. Auf diese Weise kann in praktisch quantitativer Ausbeute die Verbindung V in reiner Form erhalten werden.

In der dritten Stufe des erfindungsgemäßen Verfahrens wird die Dinitrilverbindung der Formel V in Gegenwart einer Phenylethylaminverbindung der Formel VI reduziert. Die Reduktion erfolgt vorzugsweise als katalytische Hydrierung mit einem Edelmetallkatalysator, vorzugsweise Pd/C. Bei einer bevorzugten Ausführungsform dieser Stufe wird das Dinitril V mit 0,9 bis 1,5, vorzugsweise 1,0 bis 1,1, Äquivalenten Phenylethylaminverbindung der Formel VI oder seines Salzes und 0,1 bis 10 Gew.-% Pd/C-Katalysator, bezogen auf das Amin, wobei der Katalysator 1 bis 10 Gew.-% Pd auf C enthält, bei Temperaturen zwischen 30 und 80 °C, vorzugsweise bei 60 °C, unter Normaldruck oder Überdruck bis 6 bar umgesetzt. Als Lösungsmittel werden niedrige Alkohole, Essigsäure oder aromatische Kohlenwasserstoffe, vorzugsweise niedrige aliphatische Alkohole, wie Ethanol, verwendet. Die Aufarbeitung der Reaktionsgemische erfolgt nach üblichen Methoden.

Die erhaltene Verbindung der Formel I kann erforderlichenfalls in ihr pharmakologisch annehmbares Salz umgewandelt werden.

Geeignete Säuren sind anorganische und organische Säuren. Beispiele für anorganische Säuren sind Salzsäure und Bromwasserstoffsäure. Ein Beispiel für geeignete organische Säuren ist Oxalsäure. Grundsätzlich sind alle in der Pharmazie geeigneten anorganischen und organischen Säuren für die Umwandlung in das physiologisch annehmbare Salz einsetzbar.

Das erfindungsgemäße Verfahren bietet gegenüber den bekannten Verfahren nach dem Stand der Technik die Vorteile von weniger Stufen, besserer Teil- und Gesamtausbeuten und der Verwendung weniger aggressiver und gesundheitsgefährdender Reagenzien.

Die Erfindung wird in den Beispielen erläutert.

## Beispiel 1

a) Herstellung von α-Isopropylveratrylcyanid

457,4 g (2 Mol) α-Isopropylveratrylchlorid werden mit 108 g (2,2 Mol) Natriumcyanid, 10 Mol-% Phasentransferkatalysator, 100 ml Wasser und 800 ml Triethylamin 5 Stunden bei 60 °C gerührt. Nach Zugabe von 60 ml 30%iger Natronlauge und 300 ml Wasser trennt man die organische Phase ab. Nach Abdestillieren des Triethylamins wird der Rückstand im Feinvakuum destilliert. Das übergegangene Destillat wird aus Methanol/Wasser umkristallisiert.

Ausbeute : 310 g ≙ 71 % d. Th., Schmelzbereich : 50 bis 52 °C

b) Herstellung von 4-(3,4-Dimethoxyphenyl)-4-cyano-5-methylcapronitril

438,4 g (2 Mol) α-Isopropylveratrylcyanid und 204,2 g (2,4 Mol) 3-Methoxypropionitril werden in 400 ml Dimethylformamid gelöst und mit 36 ml 5,5 molarer Natriummethylatlösung versetzt und 1/2 Stunde auf 85 °C erhitzt. Nach dem Abkühlen versetzt man das Gemisch mit Wasser, das Reaktionsprodukt kristallisiert aus. Aus Methanol/Wasser umkristallisiert, schmilzt das Produkt bei 97 °C.

Ausbeute : 506 g ≙ 92,9 % d. Th.

c) Herstellung von α-Isopropyl-α-[(N-methyl-N-homoveratryl)-aminopropyl]-3,4-dimethoxyphenylacetonitril

In einer Hydrierapparatur werden 408 g (1,5 Mol) 4-(3,4-Dimethoxyphenyl)-4-cyano-5-methylcapronitril und 70 g Pd/C, 5 %ig, in 1 700 ml Isopropanol suspendiert und die gesamte Apparatur mit $N_2$ gespült. Nach Zugabe von 20 ml Dehyquart A® (quartäre Ammonium- oder Pyridiniumverbindung, beispielsweise beschrieben in Römpps Chemie-Lexikon, 8. Auflage, Bd. 2, Seite 884) leitet man unter kräftigem Rühren einen $H_2$-Strom durch die Apparatur und füllt das Vorratsgefäß mit Wasserstoff. Dann gibt man 325 g (1,66 Mol) N-Methylhomoveratrylamin zu dem Reaktionsgemisch und hydriert bei 50 bis 60 °C unter leichtem Wasserstoffüberdruck. Nach 4 bis 6 Stunden ist die Hydrierung beendet.

Nach Abtrennung des Katalysators wird die Isopropanollösung im Vakuum eingeengt. Man nimmt den Rückstand in Essigester auf und versetzt die Lösung mit isopropanolischer HCl bis zur sauren Reaktion. Das auskristallisierte Verapamil-HCl wird abgesaugt und aus Isopropanol umkristallisiert.

Weiße Kristalle, Schmelzbereich : 139,7 bis 145 °C

Beispiel 2

Herstellung von α-Isopropyl-α-[(N-homoveratryl)-aminopropyl]-3,4-dimethoxyphenylacetonitril

In einer Hydrierapparatur werden 272 g (1,0 Mol) 4-(3,4-Dimethoxyphenyl)-4-cyano-5-methylcapronitril und 45 g Pd/C, 5 %ig, in 1 100 ml Isopropanol suspendiert und die gesamte Apparatur mit Stickstoff gespült. Man gibt 20 ml Dehyquart A® zu, leitet unter kräftigem Rühren einen $H_2$-Strom durch die Apparatur und füllt das Vorratsgefäß mit Wasserstoff. Nach Zugabe von 200 g (1,1 Mol) Homoveratrylamin hydriert man unter leichtem Wasserstoffüberdruck 4 bis 5 Stunden bei 50 bis 60 °C.

Nach Abtrennung des Katalysators wird die Isopropanollösung im Vakuum eingeengt. Man nimmt den öligen Rückstand in 600 ml Methylenchlorid auf, wäscht die Lösung einmal mit 600 ml 2N HCl, dann mit 300 ml Wasser und zieht das Lösungsmittel im Vakuum ab. Der Rückstand wird in 800 ml Essigester in der Wärme gelöst. Das auskristallisierte Norverapamil-HCl wird abgesaugt und aus Isopropanol umkristallisiert.

Weiße Kristalle, Schmelzpunkt : 141,1 bis 142,1 °C, Ausbeute : 339 g ≙ 71 %.

**Patentansprüche**

1. Verfahren zur Herstellung basisch substituierter Phenylacetonitrile der allgemeinen Formel I

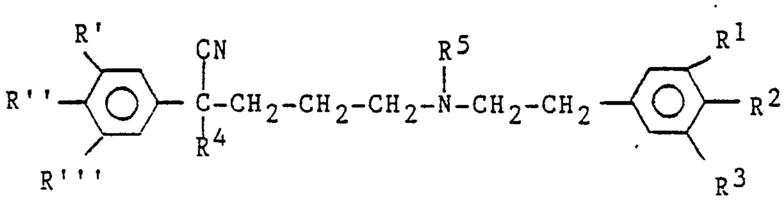

(I)

in der R', R'', R''', $R^1$, $R^2$ und $R^3$ unabhängig voneinander jeweils ein Wasserstoffatom, ein Halogenatom, eine Niedrigalkylgruppe, eine Niedrigalkoxygruppe, eine Niedrigalkylmercaptogruppe oder eine gegebenenfalls durch eine Niedrigalkylgruppe mono- oder disubstituierte Aminogruppe bedeuten, $R^4$ eine lineare oder verzweigte aliphatische Kohlenwasserstoffgruppe mit 1 bis 6 Kohlenstoffatomen, eine gesättigte oder ungesättigte cyclische Kohlenwasserstoffgruppe mit 5 oder 6 Kohlenstoffatomen bedeutet und $R^5$ für ein Wasserstoffatom oder eine Niedrigalkylgruppe steht, dadurch gekennzeichnet, daß man eine Verbindung der allgemeinen Formel II

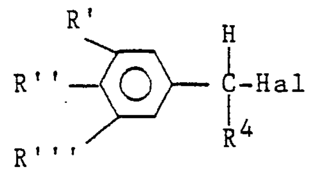

(II)

in der R', R'', R''' und $R^4$ die oben angegebenen Bedeutungen haben und Hal für ein Halogenatom steht, mit einem Cyanid der Formel Me-CN, worin Me ein einwertiges Metall bedeutet, zum Nitril der Formel III

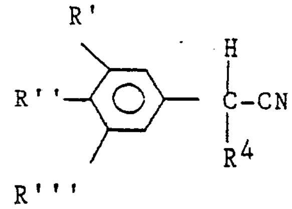

(III)

worin R', R'', R''' und $R^4$ die oben angegebenen Bedeutungen haben, umsetzt, die erhaltene Verbindung III mit einem β-substituierten Propionitril der allgemeinen Formel IV

$$NC—CH_2—CH_2—Y \quad (IV)$$

in der Y eine Niedrigalkoxygruppe oder die Gruppe NH-Z, worin Z für eine Niedrigalkylgruppe, eine $C_5$-$C_6$-Cycloakylgruppe oder einen gegebenenfalls substituierten Phenylrest steht, bedeutet, umsetzt und die erhaltene Verbindung V

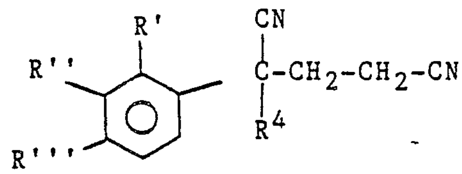

(V)

in der R', R'', R''' und $R^4$ die oben angegebenen Bedeutungen haben, in Gegenwart eines Phenylethylamins der Formel VI oder seines Salzes

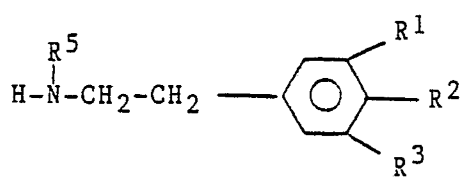

(IV)

in der $R^1$, $R^2$, $R^3$ und $R^5$ die oben angegebenen Bedeutungen haben, reduziert und gegebenenfalls die erhaltene Substanz mit einer physiologisch verträglichen Säure in ihr pharmakologisch annehmbares Salz umwandelt.

2. Verfahren nach Anspruch 1, dadurch gekennzeichnet, daß man eine Verbindung der allgemeinen Formel I herstellt, bei der R' für ein Wasserstoffatom steht, R'' und R''' jeweils für eine Methoxygruppe stehen, $R^4$ eine Isopropylgruppe darstellt, $R^5$ für eine Methylgruppe steht, $R^1$ ein Wasserstoffatom bedeutet und $R^2$ und $R^3$ jeweils für eine Methoxygruppe stehen.

3. Verfahren nach Anspruch 1 oder 2, dadurch gekennzeichnet, daß man die Reduktion in Gegenwart eines Phenylethylamins der Formel VI oder seines Salzes mit einem Edelmetall-, vorzugsweise Pd/C-, -katalysator in einer Menge von 0,1 bis 10 Gew.-%, bezogen auf die Verbindung VI, bei einer Temperatur von 30 bis 80 °C bei Normaldruck oder einem Wasserstoffdruck von bis zu 6 bar durchführt.

4. Verfahren nach einem der Ansprüche 1 bis 3, dadurch gekennzeichnet, daß man die Verbindung II in alkalischem Medium, bevorzugt Triethylamin und 1 bis 10 Gew.-% Wasser, mit Cyanid in Gegenwart eines Phasentransferkatalysators umsetzt.

5. Verfahren nach Anspruch 4, dadurch gekennzeichnet, daß man als Phasentransferkatalysator ein quaternäres Ammoniumsalz, bevorzugt Cetyltrimethylammoniumchlorid, in einer Menge von 1 bis 20 Mol-% einsetzt.

6. Verfahren nach einem der Ansprüche 1 bis 5, dadurch gekennzeichnet, daß man die Verbindung III mit 3-Methoxypropionitril in Dimethylformamid mit Natriumalkoholat bei einer Temperatur von 40 bis 90 °C umsetzt.

**Claims**

1. Process for the preparation of basic-substituted phenyl-acetonitriles corresponding to the general formula I

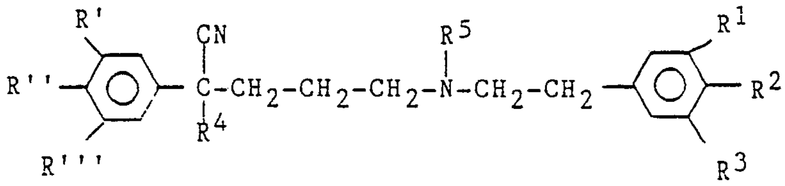

(I)

wherein R′, R″, R‴, $R^1$, $R^2$ and $R^3$ denote, independently of one another, a hydrogen atom, a halogen atom, a lower alkyl group, a lower alkoxy group, a lower alkylmercapto group or an amino group which is optionally mono- or di-substituted by a lower alkyl group, $R^4$ denotes a straight chained or branched aliphatic hydrocarbon group having 1 to 6 carbon atoms or a saturated or unsaturated cyclic hydrocarbon group having 5 or 6 carbon atoms and $R^5$ denotes a hydrogen atom or a lower alkyl group, characterised in that a compound corresponding to the general formula II

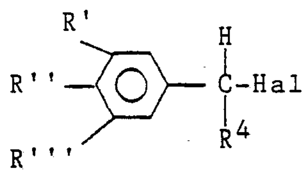

(II)

wherein R′, R″, R‴ and $R^4$ have the meanings indicated above and Hal denotes a halogen atom is reacted with a cyanide of the formula Me-CN wherein Me denotes a monovalent metal to form a nitrile corresponding to formula III

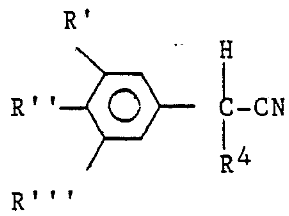

(III)

wherein R′, R″, R‴ and $R^4$ have the meanings indicated above, and the resulting compound III is reacted with a β-substituted propionitrile corresponding to the general formula IV

$$NC{-}CH_2{-}CH_2{-}Y \qquad (IV)$$

wherein Y denotes a lower alkoxy group or the group NH-Z wherein Z denotes a lower alkyl group, $C_5$ to $C_6$ cycloalkyl group or an optionally substituted phenyl group, and the resulting compound V

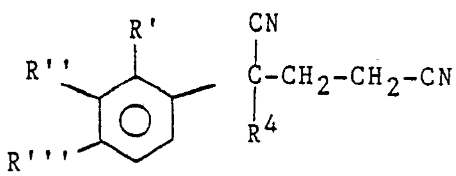

(V)

wherein R′, R″, R‴ and $R^4$ have the meanings indicated above is reduced in the presence of a phenyl ethylamine corresponding to formula VI

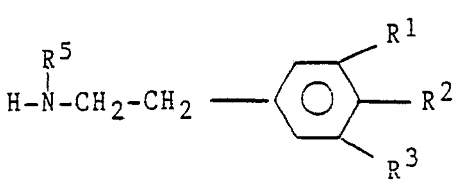

(VI)

wherein $R^1$, $R^2$, $R^3$ and $R^5$ have the meanings indicated above or a salt of said phenylethylamine, and the resulting substance is optionally converted into a pharmacologically acceptable salt thereof by reaction with a physiologically acceptable acid.

2. Process according to Claim 1, characterised in that a compound corresponding to the general formula I is prepared, in which formula, R′ stands for a hydrogen atom R″ and R‴ stand each for a methoxy

group, $R^4$ stands for an isopropyl group, $R^5$ stands for a methyl group, $R^1$ stands for a hydrogen atom and $R^2$ and $R^3$ stand each for a methoxy group.

3. Process according to Claim 1 or 2, characterised in that the reduction in the presence of a phenylethylamine of formula VI or a salt thereof is carried out with a noble metal catalyst, preferably a Pd/C catalyst, in a quantity of from 0.1 to 10 % by weight, based on compound VI, at a temperature of 30 to 80 °C and at normal pressure or a hydrogen pressure of up to 6 bar.

4. Process according to one of the Claims 1 to 3, characterised in that compound II is reacted with cyanide in an alkaline medium, preferably triethylamine and 1 to 10 % by weight of water, in the presence of a phase transfer catalyst.

5. Process according to Claim 4, characterised in that the phase transfer catalyst used is a quaternary ammonium salt, preferably cetyl trimethyl ammonium chloride, used in a quantity of from 1 to 20 mol%.

6. Process according to one of the Claims 1 to 5, characterised in that compound III is reacted with 3-methoxy-propionitrile in dimethylformamide with sodium alcoholate at a temperature of from 40 to 90 °C.

**Revendications**

1. Procédé de préparation de phénylacétonitrile substitué par un radical basique, de formule générale I

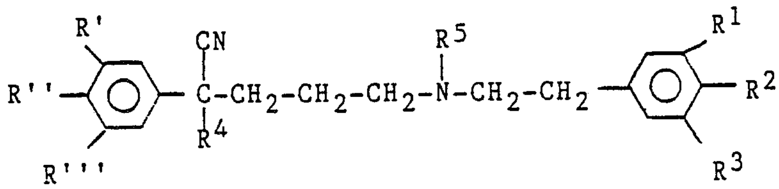

(I)

dans laquelle R′, R″, R‴, $R^1$, $R^2$ et $R^3$ désignent chacun, indépendamment les uns des autres, un atome d'hydrogène, un atome d'halogène, un groupe alcoyle inférieur, un groupe alcoxy inférieur un groupe alcoyl(inférieur) mercapto ou un groupe amino éventuellement mono- ou di-substitué par un groupe alcoyle inférieur, $R^4$ désigne un groupe hydrocarboné aliphatique linéaire ou ramifié ayant 1 à 6 atomes de carbone, un groupe hydrocarboné cyclique saturé ou insaturé ayant 5 ou 6 atomes de carbone et $R^5$ représente un atome d'hydrogène ou un groupe alcoyle inférieur, caractérisé en ce qu'on fait réagir un composé de formule générale II

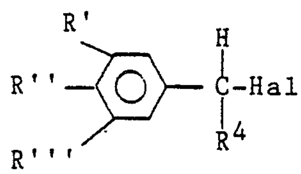

(II)

dans laquelle R′, R″, R‴ et $R^4$ sont définis comme spécifié ci-dessus et Hal représente un atome d'halogène, avec un cyanure de formule Me-CN, dans laquelle Me désigne un métal monovalent, en ce que l'on obtient un nitrile de formule III

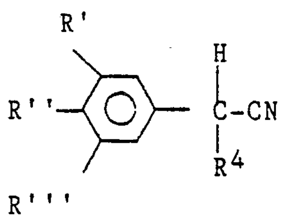

(III)

dans laquelle R′, R″, R‴ et $R^4$ sont définis comme spécifié ci-dessus, en ce que l'on fait réagir le composé III obtenu avec un propionitrile β-substitué de formule générale IV

$$NC—CH_2—CH_2—Y \qquad (IV)$$

dans laquelle Y désigne un groupe alcoxy inférieur ou le groupe NH-Z, dans lequel Z représente un groupe alcoyle inférieur, un groupe cycloalcoyle en $C_5$-$C_6$ ou un radical phényle éventuellement substitué, en ce que l'on réduit le composé V obtenu

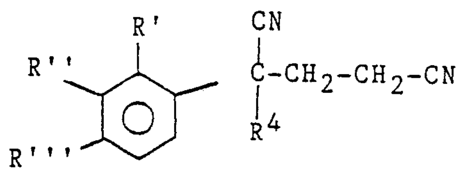

(V)

dans laquelle R', R'', R''' et $R^4$ sont définis comme spécifié ci-dessus, en présence d'une phényléthylamine de formule VI ou son sel

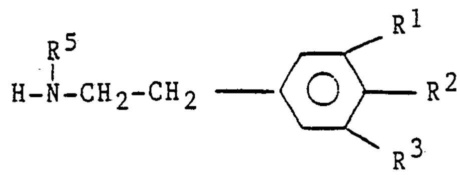

(VI)

dans laquelle formule $R^1$, $R^2$, $R^3$ et $R^5$ sont définis comme spécifié ci-dessus, et en ce qu'éventuellement on transforme la substance obtenue avec un acide physiologiquement compatible en son sel pharmacologiquement acceptable.

2. Procédé selon la Revendication 1, caractérisé en ce qu'on prépare un composé de formule générale I dans laquelle R' représente un atome d'hydrogène, R'' et R''' représentent chacun un groupe méthoxy, $R^4$ représente un groupe isopropyle, $R^5$ représente un groupe méthyle, $R^1$ désigne un atome d'hydrogène et $R^2$ ainsi que $R^3$ représentent chacun un groupe méthoxy.

3. Procédé selon la Revendication 1 ou 2, caractérisé en ce qu'on effectue la réduction en présence d'une phényléthylamine de formule VI ou de son sel avec un catalyseur à base d'un métal noble, de préférence un catalyseur à base de Pd/C, en une quantité de 0,1 à 10 % en poids par rapport au composé VI, à une température de 30 à 80 °C à la pression normale ou une pression d'hydrogène pouvant s'élever jusqu'à 6 bars.

4. Procédé selon l'une des Revendications 1 à 3, caractérisé en ce qu'on fait réagir le composé II avec le cyanure dans un milieu alcalin, de préférence la triéthylamine et 1 à 10 % en poids d'eau, en présence d'un catalyseur à transfert de phase.

5. Procédé selon la Revendication 4, caractérisé en ce qu'on utilise comme catalyseur à transfert de phase un sel d'ammonium quaternaire, de préférence le chlorure de cétyltriméthylammonium, en une quantité de 1 à 20 % en moles.

6. Procédé selon l'une des Revendications 1 à 5, caractérisé en ce qu'on fait réagir le composé III avec le 3-méthoxypropionitrile dans du diméthylformamide en présence d'un alcoolate de sodium à une température de 40 à 90 °C.